# EUROPEAN PATENT APPLICATION

(11) **EP 4 814 098 A1**
(43) Date of publication of application: **30.09.2026**
(21) Application number: 24894130.4
(22) Date of filing: 19.11.2024
(51) Int. Cl.: C12M 1/34, C12Q 1/32, G01N 33/64

(54) **DRY ANALYTICAL ELEMENT FOR TOTAL KETONE BODY ANALYSIS AND METHOD FOR MEASURING TOTAL KETONE BODIES**

(30) Priority: 20.11.2023 JP 2023196335
(71) Applicant: FUJIFILM Corporation, Tokyo 106-8620 (JP)
(72) Inventor: ABE Yoshihiko, Ashigarakami-gun, Kanagawa 258-8538 (JP); ITAI Tomokazu, Ashigarakami-gun, Kanagawa 258-8538 (JP)
(74) Representative: Dehns Germany Partnerschaft mbB
(86) International application number: PCT/JP2024/040896
(87) International publication number: WO 2025/110139

(57) **Abstract**

An object of the present invention is to provide a dry analytical element for total ketone body analysis, which is simple and inexpensive and can be used as a quantitative reagent for total ketone body amount, and a method for measuring total ketone bodies using the dry analytical element for total ketone body analysis. According to the present invention, there is provided a dry analytical element for total ketone body analysis, including, on a support in the following order, at least one water-soluble polymer layer and at least one spreading layer, in which at least one of the water-soluble polymer layer or the spreading layer contains 3-hydroxybutyrate dehydrogenase, a thionicotinamide coenzyme, a reduced nicotinamide coenzyme, and a buffer.

## Description

### Technical Field

The present invention relates to a dry analytical element for total ketone body analysis and a method for measuring total ketone bodies, which use 3-hydroxybutyrate dehydrogenase, thionicotinamide coenzyme, and reduced nicotinamide coenzyme.

### Background Art

In general, in a living body, energy is usually obtained from glucose metabolism using glucose, but in a case of diabetes or a starvation state, which is abnormal glucose metabolism, another substance called a ketone body is used as an alternative energy. The ketone body is synthesized from acetyl CoA in the liver. The ketone body includes three types of acetone, acetoacetic acid (AcAc), and 3-hydroxybutyric acid (3-HB), and these are collectively referred to as "ketone body". Among these, acetone is volatile and easily exhaled, and thus the total amount of acetoacetic acid and 3-hydroxybutyric acid present in the blood is measured as "total ketone body".

In a state in which the total ketone body increases, the energy metabolism is biased toward fatty acids, and the amount of the total ketone body is useful as an indicator of metabolism. In a case where the total ketone body in the blood increases due to a lack of sugar caused by abnormal glucose metabolism, the ketone body is acidic, and thus the pH of the blood is biased toward acidity, resulting in so-called ketoacidosis. In a case of ketoacidosis, dehydration, central nervous system disorders, coma, or the like may occur, which may lead to death. Even in this sense, it is required to immediately and accurately measure the total ketone body as monitoring of energy restriction.

As a method for measuring the total ketone body in a solution (solution method), a method for performing measurement using enzymatic cycling is known. In Patent Document 1 and Patent Document 2, a method for measuring the total ketone body amount by reacting reduced nicotinamide coenzymes (hereinafter, referred to as "NADHs") and thionicotinamide coenzymes (thio-NAD) (or thio-NADP) as coenzymes and measuring the amount of thio-NADHs to be generated is disclosed.

### Prior Art Documents

### Patent Documents

Patent Document 1: JP-H4-158799A
Patent Document 2: JP-H6-253895A

### Summary of Invention

### Object to be solved by the invention

A measurement reagent for the total ketone body has been established as a reagent to be measured by a large automatic analyzer. However, it is known that the value of the blood ketone body decreases as time elapses until the measurement, such as transportation to a testing center, and thus it is necessary to immediately perform blood plasma separation after blood collection to measure the value of the blood ketone body, and a reagent and a measurement system that can be immediately measured after blood collection are desired.

An object of the present invention is to provide a dry analytical element for total ketone body analysis, which can be used as a reagent for immediately quantifying a blood total ketone body amount after blood collection, and a method for measuring a total ketone body using the dry analytical element for total ketone body analysis.

### Means for solving the object

As a result of intensive studies to achieve the above-described object, the present inventors have found that, by a dry analytical element for total ketone body analysis in which at least one water-soluble polymer layer and at least one spreading layer are provided in this order on a support, and at least one of the water-soluble polymer layer or the spreading layer contains 3-hydroxybutyrate dehydrogenase, thionicotinamide coenzyme (thio-NAD), reduced nicotinamide coenzyme (NADH), and a buffer, a dry chemistry reagent that is simple and does not require water supply and drainage equipment or the like, and can be used as a reagent for immediately quantifying a total ketone body amount after blood collection and blood plasma separation can be provided. The present invention has been completed based on the above findings. According to an aspect of the present invention, the following inventions are provided.
<1> A dry analytical element for total ketone body analysis, comprising, on a support in the following order, at least one water-soluble polymer layer and at least one spreading layer,
   in which at least one of the water-soluble polymer layer or the spreading layer contains 3-hydroxybutyrate dehydrogenase, thionicotinamide coenzyme, reduced nicotinamide coenzyme, and a buffer.
<2> The dry analytical element for total ketone body analysis according to <1>, in which the water-soluble polymer layer is a gelatin layer.
<3> The dry analytical element for total ketone body analysis according to <1> or <2>, in which the buffer is a buffer having a buffering capacity in a region of a pH of 6.0 to 10.0.
<4> The dry analytical element for total ketone body analysis according to any one of <1> to <3>, in which the buffer is 4-(2-hydroxyethyl)-1-piperazineethanesulfonic acid, 2-hydroxy-3-morpholinopropanesulfonic acid, or 2-[N,N-bis(2-hydroxyethyl)amino]-1-ethanesulfonic acid.
<5> The dry analytical element for total ketone body analysis according to any one of <1> to <4>, in which the thionicotinamide coenzyme is thio-NAD, and the reduced nicotinamide coenzyme is NADH.
<6> The dry analytical element for total ketone body analysis according to any one of <1> to <5>, in which a content of the thionicotinamide coenzyme is 0.6 to 2.6 g/m².
<7> The dry analytical element for total ketone body analysis according to any one of <1> to <6>, in which a content of the reduced nicotinamide coenzyme is 0.4 to 1.80 g/m².
<8> The dry analytical element for total ketone body analysis according to any one of <1> to <7>, in which a content of the 3-hydroxybutyrate dehydrogenase is 1,000 to 8,000 KU/m².
<9> A method for measuring total ketone bodies, comprising: spotting a sample on the dry analytical element for total ketone body analysis according to any one of <1> to <8>; and measuring color development.

### Effect of the invention

According to the dry analytical element for total ketone body analysis and the method for measuring total ketone bodies of the present invention, it is possible to provide a dry chemistry reagent that does not require water supply and drainage equipment and can be used as a quantitative reagent for the total amount of ketone bodies.

### BRIEF DESCRIPTION OF THE DRAWINGS

[FIG. 1] FIG. 1 shows a relationship between a ΔOD/min value and a total ketone body concentration in Example 1.
[FIG. 2] FIG. 2 shows a relationship between a ΔOD/min value and a total ketone body concentration in Example 2.
[FIG. 3] FIG. 3 shows a relationship between a ΔOD/min value and a total ketone body concentration in Example 3.
[FIG. 4] FIG. 4 shows a relationship between a ΔOD/min value and a total ketone body concentration in Example 4.
[FIG. 5] FIG. 5 shows a relationship between a ΔOD/min value and a total ketone body concentration in Example 5.

### Embodiments for carrying out the invention

Hereinafter, embodiments of the present invention will be specifically described.

In the present specification, the numerical range indicated by using "to" means a range that includes numerical values described before and after "to" as a minimum value and a maximum value, respectively.

The present invention relates to a dry analytical element for total ketone body analysis, including, on a support in the following order, at least one water-soluble polymer layer and at least one spreading layer, in which at least one of the water-soluble polymer layer or the spreading layer contains 3-hydroxybutyrate dehydrogenase, thionicotinamide coenzyme, reduced nicotinamide coenzyme, and a buffer.

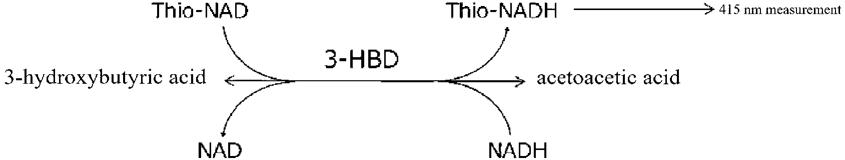

The method according to the embodiment of the present invention uses the above-described reaction system in which 3-hydroxybutyrate dehydrogenase (3-HBD) acts as a catalyst. In a case where 3-hydroxybutyric acid (3-HB) is present in a sample, 3-hydroxybutyric acid is specifically oxidized by 3-hydroxybutyrate dehydrogenase (3-HBD) in the presence of thionicotinamide coenzyme (thio-NAD) to generate acetoacetic acid (AcAc) and reduced thionicotinamide coenzyme (thio-NADH). On the other hand, acetoacetic acid (AcAc) is also specifically reduced by 3-hydroxybutyrate dehydrogenase (3-HBD) in the presence of reduced nicotinamide coenzyme (NADH) to generate 3-hydroxybutyric acid (3-HB) and nicotinamide coenzyme (NAD). In this way, 3-hydroxybutyric acid (3-HB), which is a substrate of 3-hydroxybutyrate dehydrogenase (3-HBD), and acetoacetic acid (AcAc) are subjected to enzyme cycling, and the generation rate or the generation amount of reduced thionicotinamide coenzyme (thio-NADH) generated in this way is measured, whereby it is possible to measure the total ketone body amount which is the sum of 3-hydroxybutyric acid (3-HB) and acetoacetic acid (AcAc) in the sample.

The water-soluble polymer layer used in the present invention represents a layer containing a water-soluble polymer, which is provided on a support. The water-soluble polymer layer plays a role of substantially uniformly spreading a specimen in the analytical element by drawing the specimen containing total ketone bodies, which is spotted on a spreading layer described later, into the water-soluble polymer layer. As a preferred water-soluble polymer contained in the water-soluble polymer layer, gelatin is preferable. That is, the water-soluble polymer layer is preferably a gelatin layer. The water-soluble polymer has a particularly swelling performance. The amount of the water-soluble polymer used is not particularly limited, but is preferably 8.0 g/m² or more and 40.0 g/m² or less, and more preferably 15.0 g/m² or more and 30.0 g/m² or less.

The spreading layer used in the present invention is a layer having an action (metering function) of spreading an aqueous liquid sample, which is spotted and supplied to an upper surface of a dry analytical element for total ketone body analysis, in a lateral direction without substantially unevenly distributing components contained in the aqueous liquid sample, and supplying the aqueous liquid sample to a lower layer containing a water-absorbent water-soluble polymer at a substantially constant capacity per unit area.

As the spreading layer, for example, a woven spreading layer (for example, a plain woven fabric such as broadcloth or poplin) described in JP1980-164356A (JP-S55-164356A), JP1982-66359A (JP-S57-66359A), or the like; a knitted spreading layer (for example, a tricot knitted fabric, a double tricot knitted fabric, a Milanese knitted fabric, or the like) described in JP1985-222769A (JP-S60-222769A) or the like; a spreading layer consisting of wet-laid paper containing organic polymer fiber pulp as described in JP1982-148250A (JP-S57-148250A); a nonfibrous isotropic porous spreading layer such as a membrane filter (brush polymer layer) as described in JP1978-21677A (JP-S53-21677A) and US3992158A, or a continuous microporous porous layer in which polymer microbeads, glass microbeads, or diatomaceous earth are held by a water-soluble polymer binder; a nonfibrous isotropic porous spreading layer composed of a continuous microporous porous layer (three-dimensional lattice-like particulate structure layer) in which polymer microbeads are bonded in point contact with each other by a polymer adhesive that does not swell in water as described in JP1980-90859A (JP-S55-90859A); or the like can be used. As the spreading layer, a knitted spreading layer (for example, a tricot knitted fabric, a double tricot knitted fabric, a Milanese knitted fabric, or the like) is preferable.

In addition, an interlayer such as an adhesive layer can also be provided between the support and the layer provided thereon and between the respective layers provided on the support.

As the support, a water-impermeable support is preferable. As the material of the water-impermeable support, a polymer such as polyethylene terephthalate, polycarbonate of bisphenol A, polystyrene, or cellulose ester (for example, cellulose diacetate, cellulose triacetate, cellulose acetate propionate, or the like) is preferable, and polyethylene terephthalate is particularly preferable. As the support, a smooth planar support that is transparent, for example, transmits electromagnetic radiation having a wavelength in a range of at least a part of a wavelength range of approximately 200 nm to approximately 900 nm, in a thickness range of approximately 50 µm to approximately 1 mm and preferably approximately 80 µm to approximately 300 µm can be used. A known undercoat or adhesive layer can be provided on a surface of the support to firmly adhere the interlayer.

In the dry analytical element for total ketone body analysis according to the embodiment of the present invention, at least one of the water-soluble polymer layer or the spreading layer contains 3-hydroxybutyrate dehydrogenase (3-HBD). The 3-hydroxybutyrate dehydrogenase is an enzyme that oxidizes 3-hydroxybutyric acid, and is a conjugate enzyme that reduces acetoacetic acid to catalyze the generation of 3-hydroxybutyric acid. As a content of the 3-hydroxybutyrate dehydrogenase (3-HBD), in order to ensure sensitivity, 1,000 to 8,000 KU/m² is preferable, and 3,000 to 5,000 KU/m² is more preferable. Here, 1 U is defined as an enzyme amount capable of changing 1 µmol of 3-hydroxybutyric acid to acetoacetic acid per minute under optimum conditions.

The dry analytical element for total ketone body analysis according to the embodiment of the present invention contains a (oxidized) thionicotinamide coenzyme. The (oxidized) thionicotinamide coenzyme is a coenzyme that works by binding to various oxidoreductases in the same manner as the nicotinamide coenzyme, and is involved in hydrogen transfer in vivo, and specifically means thio-NAD or thio-NADP. The (oxidized) thionicotinamide coenzyme is preferably thio-NAD. As a content of the (oxidized) thionicotinamide coenzyme, in order to ensure sensitivity, 0.6 to 2.6 g/m² is preferable, 0.8 to 2.0 g/m² is more preferable, and 0.9 to 1.7 g/m² is still more preferable. In a case where the content of the (oxidized) thionicotinamide coenzyme is 1.5 g/m² or less, the background is reduced, which is preferable.

The dry analytical element for total ketone body analysis according to the embodiment of the present invention contains a reduced nicotinamide coenzyme. The reduced nicotinamide coenzyme is a coenzyme of various dehydrogenases, and specifically means NADH or NADPH. The reduced nicotinamide coenzyme is preferably NADH. As an amount of the reduced nicotinamide coenzyme used, in order to ensure sensitivity, 0.4 to 1.8 g/m² is preferable, 0.5 to 1.6 g/m² is more preferable, and 0.6 to 1.2 g/m² is still more preferable. In a case where the content of the reduced nicotinamide coenzyme is 1.8 g/m² or less, the background is reduced, which is preferable.

The dry analytical element for total ketone body analysis according to the embodiment of the present invention contains a buffer.

The buffer is preferably a buffer having a buffering capacity in a region of pH 5.0 to 9.0, and more preferably a buffer having a buffering capacity in a region of pH 6.0 to 8.0.

Examples of the type of the buffer include known buffers such as tris(hydroxymethyl)aminomethane and Good's buffer. The buffer is preferably 4-(2-hydroxyethyl)-1-piperazineethanesulfonic acid (also referred to as HEPES), 2-hydroxy-3-morpholinopropanesulfonic acid (also referred to as MOPSO), or 2-[N,N-bis(2-hydroxyethyl)amino]-1-ethanesulfonic acid (also referred to as BES). The buffer is more preferably 4-(2-hydroxyethyl)-1-piperazineethanesulfonic acid (HEPES).

The content of the buffer is not particularly limited as long as the pH of the specimen is not affected, but is preferably 1.0 to 10.0 g/m² and more preferably 3.0 to 8.0 g/m².

In the present invention, the quantitative determination of the total ketone body amount is possible by containing the 3-hydroxybutyrate dehydrogenase (3-HBD), the thionicotinamide coenzyme, the reduced nicotinamide coenzyme, and the buffer in at least one of the water-soluble polymer layer or the spreading layer. That is, the 3-hydroxybutyrate dehydrogenase, the (oxidized) thionicotinamide coenzyme, the reduced nicotinamide coenzyme, and the buffer may be contained in the water-soluble polymer layer, may be contained in the spreading layer, or may be contained in both the water-soluble polymer layer and the spreading layer. More preferably, a configuration in which these are contained in the spreading layer is preferable.

The dry analytical element for total ketone body analysis according to the embodiment of the present invention may be an analytical element further including a reagent layer, a reflective layer, a light cut layer, a filtration layer, an undercoat layer, and other layers. Examples of such an analytical element include those disclosed in US3992158A and US4042335A. As a preferred configuration of the present invention, an integrated multilayer analytical element produced by sequentially laminating and integrating a water-soluble polymer layer having a water absorption function and a spreading layer that spreads the specimen in the lateral direction on a light-transmitting and water-impermeable support is preferable.

The dry analytical element for total ketone body analysis according to the embodiment of the present invention can be prepared by a method known to those skilled in the art. For example, a coating liquid prepared as an interlayer coating liquid is applied onto the support and dried to produce a dried film having a thickness of about 40 µm, and then a woven fabric of the spreading layer is bonded thereto. Thereafter, a dry analytical element for total ketone body analysis can be produced by applying a coating liquid prepared as a reagent holding layer solution from the woven fabric side of the spreading layer and drying the coating liquid. From the viewpoint of manufacturing, packaging, transportation, storage, measurement operation, and the like, it is preferable that the dry analytical element for total ketone body analysis is cut into a small piece having a square shape with a side of about 15 mm to about 30 mm, a shape of the same size as the square shape, or the like, and is used as a chemical analysis slide by being housed in a slide frame described in JP1982-28331B (JP-S57-28331B), JP1981-142454U (JP-S56-142454U), JP1982-63452A (JP-S57-63452A), JP1983-32350U (JP-S58-32350U), JP1983-501144A (JP-S58-501144A), and the like. Depending on the purpose of use, the dry analytical element for total ketone body analysis can be used in a long tape shape housed in a cassette or a magazine, or can be used by being attached to a card having an opening or housed in the card.

According to the present invention, there is provided a method for measuring total ketone bodies, including spotting a sample on the dry analytical element for total ketone body analysis according to the embodiment of the present invention and measuring color development. The sample may be a sample containing at least one of 3-hydroxybutyric acid or acetoacetic acid.

For example, an aqueous liquid sample such as whole blood, blood plasma, serum, lymph, or urine in a range of about 5 µL to about 30 µL, preferably about 8 µL to about 15 µL is spotted on the spreading layer, and incubated at a substantially constant temperature in a range of about 20°C to about 40°C, preferably at a substantially constant temperature in the vicinity of 37°C for about 1 minute to about 10 minutes, preferably in a range of about 2 minutes to about 7 minutes, and a change that can be detected such as a color change or color development in the dry analytical element for total ketone body analysis is subjected to reflectance photometry from the support side, whereby the content of total ketone bodies in the liquid sample can be determined according to the principle of the colorimetric method. In the present invention, the optical density of the spreading layer is subjected to reflectance photometry using light having an absorption maximal wavelength of color development by total ketone bodies or a wavelength in the vicinity of the absorption maximal wavelength, and the content of total ketone bodies in the liquid sample can be determined according to the principle of the colorimetric method using a calibration curve prepared in advance. By setting the amount of the aqueous liquid sample to be spotted, the incubation time, and the incubation temperature to be constant, quantitative analysis of total ketone bodies can be performed with high accuracy. In the measurement operation, it is possible to perform a highly accurate quantitative analysis by a chemical analysis apparatus described in JP1985-125543A (JP-S60-125543A), JP1985-220862A (JP-S60-220862A), JP1986-294367A (JP-S61-294367A), JP1983-161867A (JP-S58-161867A), and the like in an extremely easy operation.

Next, the present invention will be described using Examples, but the present invention is not limited thereto.

### Examples

### <Example 1>

### (1) Production of coating film and dry analytical slide

A coating film was produced by applying an aqueous solution of the following composition-1 to a smooth 180 µm colorless transparent polyethylene terephthalate (PET) film which had been coated with gelatin as an undercoat, such that the thickness after drying was 40 µm, and drying the applied aqueous solution to provide a water-absorbing layer.

### Water-absorbing layer (composition-1)

Gelatin 17 g/m²
Surfactant 0.2 g/m²

Here, as the surfactant, polyoxy(2-hydroxy)propylene nonylphenyl ether (Surfactant 10G, manufactured by Olin Corporation) was used.

Next, water was supplied to the front surface of the film at a supply amount of about 30 g/m² to wet the film, and then a polyester spun yarn tricot knitted fabric having a denier of 50 was bonded thereto using a wet lamination method while applying a light pressure, to provide a spreading layer.

Next, an aqueous solution A having the following composition was applied onto the spreading layer such that each component was present in the following amount, and the applied aqueous solution was dried to produce a dry analytical element for total ketone body analysis according to the embodiment of the present invention.

### Aqueous solution A

3-hydroxybutyrate dehydrogenase (3-HBD) (manufactured by Asahi Kasei Pharma Corporation) 38004 KU/m²
Thio-NAD (manufactured by Oriental Yeast Co., Ltd.) 1.28 g/m²
NADH (manufactured by Oriental Yeast Co., Ltd.) 0.90 g/m²
HEPES (manufactured by Dojindo Laboratories) 4.0 g/m²
Polyvinylpyrrolidone (manufactured by BASF SE) 10.9 g/m²

The dry analytical element for total ketone body analysis was cut into a size of 12 mm × 13 mm, and a slide was prepared according to the method described in JP1982-63452A (JP-S57-63452A) to produce a dry analytical slide (1) for total ketone body analysis.

### (2) Measurement of total ketone body concentration

A human pooled serum specimen prepared such that the total ketone body concentration was 300 µmol/L and 105 µmol/L, and a 7% human serum albumin (HSA) aqueous solution as a measurement reagent having a total ketone body concentration of zero were prepared, 10 µL of each of the human pooled serum specimen and the 7% HSA aqueous solution was spotted on the dry analytical slide (1) prepared in Example 1, and the reflection density at 415 nm was measured every 10 seconds for 3 minutes by a FUJIFILM DryChem 7000 analyzer (manufactured by FUJIFILM Corporation) while maintaining the temperature at 37°C.

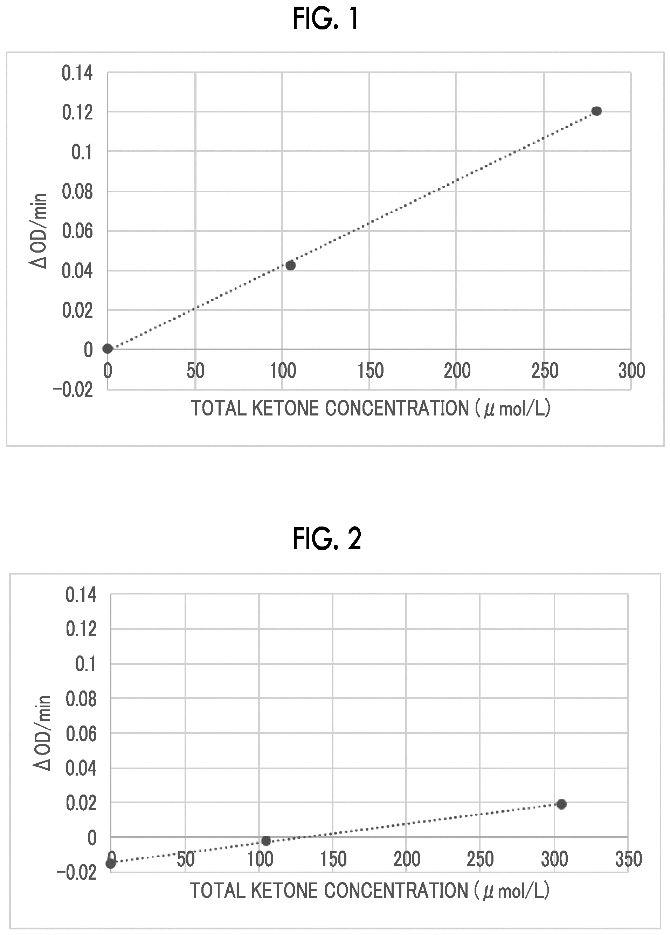

The concentration of the total ketone body and the change amount (ΔOD/min) of the reflection density per minute in the reflection density that increased in a measurement time of 60 sec to 180 sec are summarized in Table 1, and the relationship between the concentration of the total ketone body and the ΔOD/min value with respect to the total ketone body concentration, in which the concentration of the total ketone body is taken as the horizontal axis and the ΔOD value per minute is taken as the vertical axis, is shown in FIG. 1.

**[Table 1]**

| Total ketone body (µmol/L) | ΔOD/min |
|---|---|
| 0.0 | 0.0005 |
| 105.1 | 0.0427 |
| 300.7 | 0.1205 |

### <Example 2>

### (1) Production of coating film and dry analytical slide

A smooth 180 µm colorless transparent PET film subjected to gelatin undercoating was coated with an aqueous solution of the following composition-2 such that the thickness after drying was 40 µm, and dried to provide a water-absorbing layer.

### Water-absorbing layer (composition-2)

Polyvinyl alcohol 23 g/m²
Surfactant 0.2 g/m²

Here, as the surfactant, polyoxy(2-hydroxy)propylene nonylphenyl ether (Surfactant 10G, manufactured by Olin Corporation) was used.

A dry analytical slide (2) for total ketone body analysis was produced in the same manner as in Example 1, except that the water-soluble polymer of the water-absorbing layer was changed from gelatin to polyvinyl alcohol and the water-absorbing layer was provided on the PET film as described above.

### (2) Measurement of total ketone body concentration

The measurement of the total ketone body concentration was performed in the same manner as in Example 1, and the concentration of the total ketone body and the change amount (ΔOD/min) of the reflection density per minute in the reflection density that increased in a measurement time of 60 sec to 180 sec are summarized in Table 2, and the relationship between the ΔOD/min value and the total ketone body concentration, in which the concentration of the total ketone body is taken as the horizontal axis and the ΔOD value per minute is taken as the vertical axis, is shown in FIG. 2.

**[Table 2]**

| Total ketone body (µmol/L) | ΔOD/min |
|---|---|
| 0 | -0.0152 |
| 105.1 | -0.0019 |
| 300.7 | 0.0111 |

From the results of Examples 1 and 2, it was found that both the dry analytical elements for total ketone body analysis had good linearity and exhibited sufficient performance as a test agent capable of quantitatively measuring the total ketone body. It is more preferable to use gelatin as the water-absorbing layer in the dry analytical element from the viewpoint that a larger signal-to-noise ratio (S/N) can be obtained.

### <Examples 3 to 5>

The following levels were prepared as buffers, and the prepared buffers were slide-formed and checked.

A re-experiment (Example 3) using the aqueous solution A of Example 1 and a change of HEPES in Example 3 to MOPSO (Example 4) and to BES (Example 5) were performed to produce dry analytical slides in the same manner as in Example 1. In the same manner as in Example 1, a human pooled serum specimen was prepared such that the total ketone body concentration (solution method measurement value) was 0 µmol/L, 105 µmol/L, or 300 µmol/L, 10 µL of the specimen was spotted on each of the dry analytical slides using the above-described buffer solution, and the reflection density at 415 nm was measured by a FUJIFILM DRYCHEM 7000 analyzer (manufactured by FUJIFILM Corporation) every 10 seconds for 3 minutes while maintaining the temperature at 37°C. The number of specimens was N = 2.

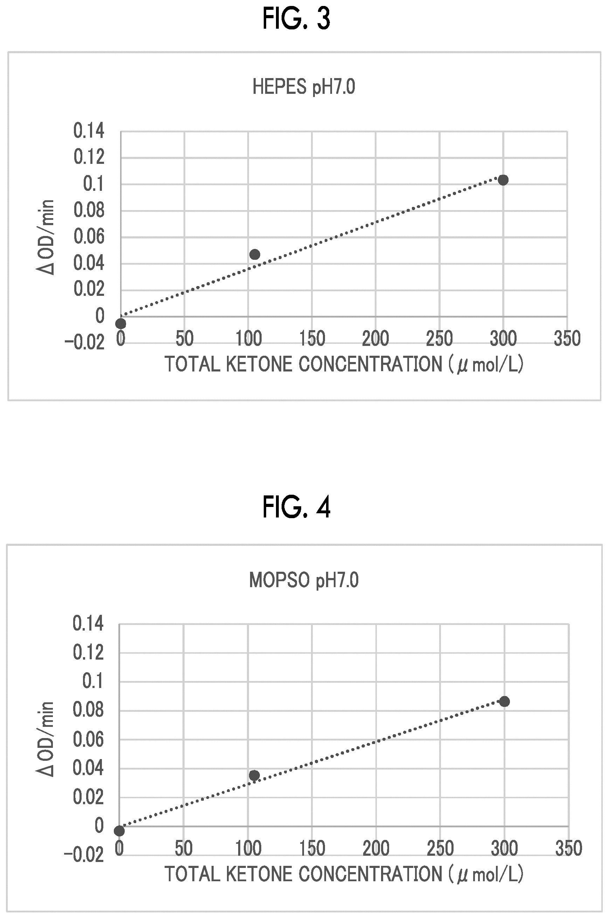

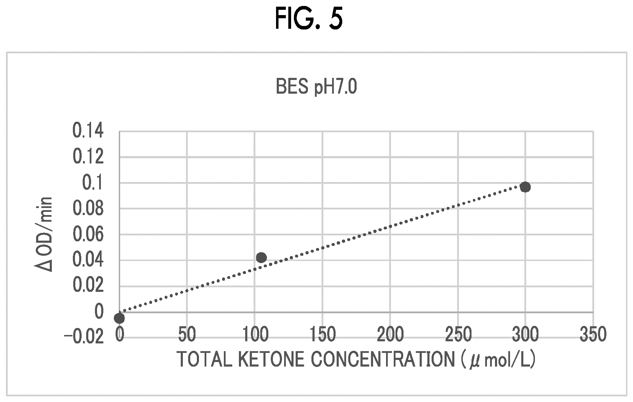

The concentration of the total ketone body and the change amount (ΔOD/min) of the reflection density per minute in the reflection density that increased in a measurement time of 60 sec to 180 sec are summarized in Table 3, and the relationship between the ΔOD/min value and the total ketone body concentration, in which the concentration of the total ketone body is taken as the horizontal axis and the ΔOD value is taken as the vertical axis, is shown in FIGS. 3 to 5.

**[Table 3]**

| | HEPES | MOPSO | BES |
|---|---|---|---|
| Total ketone body (µmol/L) | ΔOD/min | ΔOD/min | ΔOD/min |
| 0.0 | -0.0053 | -0.0033 | -0.0047 |
| 105.1 | 0.0426 | 0.0352 | 0.0420 |
| 300.7 | 0.1034 | 0.0863 | 0.0968 |

From the results of Examples 3 to 5, it was found that all of the dry analytical elements for total ketone body analysis had good linearity and exhibited sufficient performance as a test agent capable of quantitatively measuring the total ketone body.

### <Example 6>

10 µL of blood plasma (human specimen-1, human specimen-2, and human specimen-3) obtained by collecting blood and immediately performing blood plasma separation after obtaining informed consent from a volunteer patient, was quickly spotted on the dry analytical slide (1) for total ketone body analysis produced in Example 1, the change amount (ΔOD/min) of the reflection density per minute in the reflection density that increased in a measurement time of 60 sec to 180 sec was determined in the same manner as in Example 1, and the total ketone amount (µmol/L) was determined from the relationship between the ΔOD/min value and the total ketone body concentration shown in FIG. 1. The results are shown in Table 4.

In addition, the results of measuring the total ketone amount (µmol/L) of the human specimen-1, the human specimen-2, and the human specimen-3 in a solution state using a large automatic analyzer (device: BM6010 manufactured by JEOL Ltd., reagent: TKB-L manufactured by Kainos Laboratories, Inc.) are also shown in Table 4.

The reference range of the total ketone amount of a healthy subject is 26 to 122 µmol/L.

**[Table 4]**

| | Human specimen-1 | Human specimen-2 | Human specimen-3 |
|---|---|---|---|
| Drying analysis slide (1) | 29 | 48 | 74 |
| Large automatic analyzer | 29 | 50 | 65 |

As shown in Table 4, the measurement results of the total ketone amount measured using the dry analytical element for total ketone body analysis according to the embodiment of the present invention substantially matched the measurement results of the total ketone amount measured using the large automatic analyzer that measures the total ketone amount in a solution state. That is, it was confirmed that the total ketone body can be measured with high accuracy and easily immediately after blood collection by using the dry analytical element for total ketone body analysis according to the embodiment of the present invention.

## Claims

1. A dry analytical element for total ketone body analysis, comprising, on a support in the following order, at least one water-soluble polymer layer and at least one spreading layer,
wherein at least one of the water-soluble polymer layer or the spreading layer contains 3-hydroxybutyrate dehydrogenase, a thionicotinamide coenzyme, a reduced nicotinamide coenzyme, and a buffer.

2. The dry analytical element for total ketone body analysis according to claim 1,
wherein the water-soluble polymer layer is a gelatin layer.

3. The dry analytical element for total ketone body analysis according to claim 1 or 2,
wherein the buffer is a buffer having a buffering capacity in a region of pH 6.0 to 10.0.

4. The dry analytical element for total ketone body analysis according to claim 1 or 2,
wherein the buffer is 4-(2-hydroxyethyl)-1-piperazineethanesulfonic acid, 2-hydroxy-3-morpholinopropanesulfonic acid, or 2-[N,N-bis(2-hydroxyethyl)amino]-1-ethanesulfonic acid.

5. The dry analytical element for total ketone body analysis according to claim 1 or 2,
wherein the thionicotinamide coenzyme is thio-NAD, and the reduced nicotinamide coenzyme is NADH.

6. The dry analytical element for total ketone body analysis according to claim 1 or 2,
wherein a content of the thionicotinamide coenzyme is 0.6 to 2.6 g/m².

7. The dry analytical element for total ketone body analysis according to claim 1 or 2,
wherein a content of the reduced nicotinamide coenzyme is 0.4 to 1.8 g/m².

8. The dry analytical element for total ketone body analysis according to claim 1 or 2,
wherein a content of the 3-hydroxybutyrate dehydrogenase is 1,000 to 8,000 KU/m².

9. A measurement method for total ketone bodies, comprising:
spotting a sample on the dry analytical element for total ketone body analysis according to claim 1 or 2; and
measuring color development.
